# EUROPÄISCHE PATENTSCHRIFT

(11) **EP 2 004 263 B1**
(45) Veröffentlichungstag und Bekanntmachung des Hinweises auf die Patenterteilung: **29.01.2014**
(21) Anmeldenummer: 07720079.8
(22) Anmeldetag: 13.04.2007
(51) Int. Cl.: A61M 15/00

(54) **MEDIKAMENTEN-AUSGABEVORRICHTUNG UND MEDIKAMENTENMAGAZIN**
MEDICAMENT DELIVERY DEVICE AND MEDICAMENT CARRIER
DISPOSITIF D'ÉMISSION DE MÉDICAMENTS ET MAGASINS À MÉDICAMENTS

(30) Priorität: 13.04.2006 EP 06405160
(43) Veröffentlichungstag der Anmeldung: 24.12.2008
(73) Patentinhaber: BOEHRINGER INGELHEIM INTERNATIONAL GMBH, 55216 Ingelheim am Rhein (DE)
(72) Erfinder: WACHTEL, Herbert, 55218 Ingelheim am Rhein (DE); GESER, Johannes, 55216 Ingelheim am Rhein (DE); METZGER, Burkhard, P., 55218 Ingelheim am Rhein (DE); SPALLEK, Michael, 55218 Ingelheim am Rhein (DE); KRUEGER, Michael, 55218 Ingelheim am Rhein (DE); KUNZE, Hubert, 44227 Dortmund (DE); MOSER, Achim, 09130 Chemnitz (DE); MOCK, Elmar, CH-2013 Colombier (CH); LANCI, Antonino, CH-3006 Bern (CH); KLOPFENSTEIN, André, CH-2520 La Neuveville (CH)
(74) Vertreter: Frei Patent Attorneys
(86) Internationale Anmeldenummer: PCT/CH2007/000182
(87) Internationale Veröffentlichungsnummer: WO 2007/118343

(56) Entgegenhaltungen:
- WO-A-02/098495
- WO-A-03/045483
- WO-A-2007/018568
- WO-A2-01/07107
- US-A- 5 476 093
- US-A1- 2004 182 387
- US-A1- 2005 056 281

## Beschreibung

Die Erfindung liegt auf dem Gebiet von Medikamenten-Ausgabevorrichtungen mit einem Mehrdosismagazin, insbesondere von Mehrdosispulverinhalatoren und betrifft eine Medikamenten-Ausgabevorrichtung gemäss dem Oberbegriff des unabhängigen Anspruchs. Ebenfalls beansprucht ist ein Medikamentenmagazin zur Verwendung in der Medikamenten-Ausgabevorrichtung.

Aus dem Stand der Technik sind Inhalationsgeräte bekannt, in welchen durch eine beim Inhalieren erzeugte Unterdruckströmung (Venturi) direkt an eine Medikamentenkammer angeschlossen wird und ein darin befindliches pulverförmiges Medikament entnommen wird. In US 6,655,381 ist in einem Ringmagazin in kreisförmig angeordneten Vertiefungen ein pulverförmiges Medikament eingebracht. Ein die Vertiefungen schliessender Siegel wird im wesentlichen als Ganzes entfernt und ein Venturirohr mit der Verengung über der Vertiefung wird parallel zum Magazin angeordnet. An das Venturirohr angeschlossen ist eine längere Verwirbelungskammer, die nötig ist, da das Medikamente im wesentlichen auf einmal der Vertiefung entnommen wird. Eine genaue Dosierung eines Medikaments oder eine Einstellung auf unterschiedliche Dosierungen ist mit dieser Vorrichtung nicht möglich.

Zudem existieren diverse Inhalatoren, in welchen eine Medikamentenkammer durch Anstechen geöffnet wird. Öffnungen in einer Folie, welche durch ein Anstechen gemacht wurden, sind jedoch nicht sehr genau definiert, so dass zum einen die genaue entnommene Menge eines Medikaments nicht bekannt ist und zudem Unterschiede zwischen aufeinander folgenden Entnahmen resultieren. Dies ist insbesondere bei Medikamenten unerwünscht, dessen Wirkung sehr stark von einer Dosierung abhängt.

Um eine möglichst gute Verwirbelung eines Medikaments, und dadurch eine Lösung von Trägermaterial und eigentlichem Wirkstoff, zu erhalten wird in EP 1 475 115 und GB 2 340 758 eine Medikamentenkammer im wesentlichen in eine Eintritts- und eine Austrittskammer unterteilt, welche durch einen verengten Durchgang miteinander verbunden sind. Ein Medikament wird dabei nur in die Eintrittskammer eingebracht. Durch ein Anstechen bzw. Einführen von Kanülen wird ein Luftstrom durch die Medikamentenkammer von der Eintritts- über die Verengung in die Austrittskammer und aus der Medikamentenkammer hinaus bewirkt. In EP 1 475 115 wird zur besseren Entleerung der Medikamentenkammer die Eintritts- und Austrittskammer mit je zwei Öffnungen versehen. In beiden Schriften werde die Öffnungen der Kammern durch das Anstechen definiert.

In US 5,715,810 wird eine in einem Ringmagazin eingebrachte Medikamentenkammer beidseitig angestochen. Die eine Anstechkanüle ist an eine Venturiströmung angeschlossen, so dass ein Luftstrom durch die Kammer erzeugt wird. Die Entnahme erfolgt dabei in einem Schritt und kann nicht oder nur schlecht dosiert werden. Auch hier werden die Kammern durch ein Anstechen und die dabei verwendeten Nadeln definiert.

In WO 03/045483 wird eine Medikamentenkammer angestochen und ein darin befindliches Medikament mit Hilfe eines durch eine Druckluftquelle erzeugten Luftstroms entleert.

EP 0 547 429 offenbart ein zylindrisches Medikamentenmagazin, bei dem durch eine Schraubbewegung eine frische Medikamentenkammer mit einer Entnahmeöffnung im Innern der Vorrichtung in Übereinstimmung gebracht wird. In der Vorrichtung wird der Luftstrom unterteilt: während ein Teil die Medikamentenkammer leert wird der andere Teil durch die Vorrichtung geführt und als Ummantelungs-Luftstrom mit dem Medikamentenluftstrom vereint.

In WO 2005/002654 wird eine Luftströmung durch eine Medikamentenkammer injiziert. Dabei sind einzelne Medikamententaschen in einem Ringmagazin untergebracht. Die Taschen werden bei einem Gebrauch von hinten aus dem Magazin gestossen, durchtrennen dabei eine Siegelfolie und werden mit einem Entnahmemechanismus gekoppelt. Dabei wir ein Luftstrom mit möglichst hoher Geschwindigkeit in und durch die Tasche gelenkt. Um eine Aggregation eines Pulvers aufzulösen und eine Verschleppung von Medikament auf folgende Inhalationen zu vermeiden, ist jeder Tasche ein individueller Kanal nachgeschaltet. Mit einem zusätzlichen Bypasskanal, der die Tasche umgeht und ebenfalls in ein Mundstück mündet, wird das inhalierte Gesamtluftvolumen erhöht und eine Inhalation erleichtert bzw. garantiert, dass ein genügender Luftstrom vorhanden ist, um das Medikament bis zu einem Benutzer zu transportieren. Dadurch, dass ein Teil des Luftstromes durch den Bypasskanal gelenkt wird, wird der Luftstrom durch die Tasche verringert, so dass die Dauer des Entnahmevorganges verlängert wird. Die Entnahme des Medikament kann damit über eine etwas längere Zeitdauer verteilt werden anstatt alles auf einmal zu entnehmen.

Der Pulverinhalator aus WO 2005/002654 ist relativ kompliziert aufgebaut. Es sind mehrere unterschiedliche Teile vorhanden, welche teilweise bewegt und aufeinander abgestimmt werden müssen. Die Entnahme des Medikaments wird zudem nicht durch ein eigentliches Venturi-Prinzip ausgelöst, sondern es wird direkt eine Saugströmung durch die Medikamententaschen erzeugt, welche durch eine Bypassströmung abgeschwächt werden kann.

Die WO 01/07107 A2 beschreibt einen Pulver-Inhalator mit einem Einlassabschnitt einem Mischabschnitt sowie einem Mundstück mit einer Öffnung. Der Mischabschnitt ist als Venturi-Kammer ausgebildet. Die Venturi-Kammer dient zum Regeln der Strömungsrate von Luft im Mischabschnitt. Ferner weist der Mischabschnitt im Bereich der engsten Stelle der Venturi-Kammer Strukturen auf, welche einen Wirbelstrom erzeugen.

Die WO 02/098495 A1 beschreibt eine Pulverausgabe-Vorrichtung mit einem Dosieraufnahmebehälter für ein Medikamentenpulver. Der Dosieraufnahmebehälter beinhaltet ein Pulverreservoir, welches eine Spulen- Hülsenanordnung umfasst. Der Dosieraufnahmebehälter ist mit einer Siegelfolie abgedeckt, welche zwecks Entnahme des Medikamentenpulvers unter Ausbildung einer Entnahmeöffnung mit entsprechenden Mitteln durchstossen werden kann.

Aufgabe der Erfindung ist es deshalb, eine Medikamenten-Ausgabevorrichtung sowie ein Medikamentenmagazin zur Verwendung in einer solchen Medikamenten-Ausgabevorrichtung zu schaffen, in welchen die Entnahme auf einem Venturiprinzip beruht und eine Entnahmemenge genau dosierbar und reproduzierbar ist.

Die Aufgabe wird durch die Medikamenten-Ausgabevorrichtung, das Medikamentenmagazin, sowie das Verfahren gelöst, wie sie in den Ansprüchen definiert sind.

Die Erfindung beruht auf dem beispielsweise aus US 6,655,381 bekannten Prinzip, eine Venturi-ähnliche Unterdruckströmung an eine Medikamentenkammer anzulegen und das darin befindliche Medikament durch den Unterdruck aus der Kammer zu saugen und mit der Strömung in Richtung eines Mundstücks zu transportieren. Dort gelangt das Medikament mit dem Luftstrom in die Lunge einer inhalierenden Person.

In der erfindungsgemässen Vorrichtung wird nun in einem Luftkanal eine Antriebsströmung erzeugt, welche aufgrund einer Verengung im Luftkanal in diesem Bereich eine Unterdruckströmung erzeugt. Der die Unterdruckströmung verursachende engste Bereich des Luftkanals ist dabei an eine Entnahmeöffnung einer Medikamentenkammer angeschlossen. Die Entnahmeöffnung steht mit einer Steueröffnung in Verbindung, so dass eine Entleerungsströmung durch die Steueröffnung via Entnahmeöffnung und durch die Medikamentenkammer gebildet wird. Die Entleerungsströmung ist dabei vor Eintritt in die Medikamentenkammer nicht mit der Antriebsströmung verbunden, vereinigt sich jedoch nach Austritt aus der Entnahmeöffnung mit der Antriebsströmung, so dass ein mit der Entleerungsströmung mitgeführtes Medikament mit der Antriebsströmung in Richtung Mundstück transportiert wird.

Die Entnahme- und Steueröffnung sind direkt in einem Medikamentenmagazin integriert. Werden die Öffnungen oder das gesamte Medikamentenmagazin beispielsweise durch Spritzgiessen aus einem Kunststoff hergestellt, so sind diese Öffnungen sehr genau definiert, wie es bei einem Erzeugen einer Öffnung beispielsweise durch Anstechen nicht möglich ist. Durch eine Integration von Entnahme- und Steueröffnung im Medikamentenmagazin sind diese beiden Öffnungen vordefiniert und sowohl vor als auch nach einem Öffnen einer Medikamentenkammer festgelegt.

Die Steuer- und Entnahmeöffnung bilden einen wesentlichen Bestandteil einer Drosselanordnung, über welche die Entleerungsströmung und damit eine Variation einer Menge pro Zeit eines entnommenen Medikaments steuerbar ist. Weitere Elemente der Drosselanordnung können mit unterschiedlicher Wirkung die innere Gestaltung der Medikamentenkammer selber, sowie die Gestaltung des an die Entnahmeöffnung anschliessenden Luftkanals, insbesondere sein Durchmesser, sein.

Eine Entnahme kann dadurch gezielt gesteuert werden, ist genau definiert und vor allem reproduzierbar. Eine Drosselwirkung und damit eine Entleerungsströmung, ist aufgrund der exakt definierten, aber variablen Elemente wie Steueröffnung, Entnahmeöffnung, innere Gestaltung der Medikamentenkammer gezielt einstellbar und auch leicht auf andere Dosierungen, Mengen und Arten von Medikamenten änderbar.

Der Inhalator kann zur Erleichterung des Inhalierens, beispielsweise bei Kindern oder einer Person mit geschwächter Lunge, noch mindestens eine Bypassöffnung ausweisen. Diese Bypassöffnung dient zur Bildung einer Bypassströmung, welche ebenfalls zum Mundstück führt und vorzugsweise nach einer Entnahmeöffnung in Richtung des Mundstücks angeordnet ist. Sie nimmt jedoch keinen direkten Einfluss auf eine definierte Abstimmung zwischen Unterdruckströmung, Entnahmeöffnung und Steueröffnung.

Eine Medikamentenkammer ist vorzugsweise so gestaltet, dass zumindest ein Teil der Entleerungsströmung über das in der Medikamentenkammer befindliche Medikament hinwegströmt. Über eine innere Gestaltung der Medikamentenkammer kann zusätzlich Einfluss auf eine Deagglomeration und Dispersion eines Medikamentes genommen werden. Beispielsweise kann durch ein Verwirbelungselement, welches vorzugsweise durch mindestens einen Teil einer Medikamentenkammerwand selber gebildet ist, zusätzliche Turbulenzen in eine Entleerungsströmung eingebracht werden. Dies kann eine Drosselwirkung erhöhen oder bei Bedarf auch senken.

In der Medikamentenkammer kann auch eine zusätzliche Unterdruckströmung, beispielsweise durch eine spezielle Gestaltung einer Kammerwand oder durch ein Verwirbelungselement, verursacht werden. Diese ist vorzugsweise auf direkter Linie zwischen Steuer- und Entnahmeöffnung angeordnet, derart, dass ein sich auf einem Boden der Medikamentenkammer befindliches Medikament aufgrund dieser zusätzlichen in der Kammer herrschenden Unterdruckströmung löst. Dies kann dadurch erreicht werden, dass beispielsweise die Medikamentenkammer selber im ganzen oder einem Teilbereich zwischen Steuer- und Entnahmeöffnung eine Art Venturirohr bildet.

Ein Medikamentenmagazin weist typischerweise zusätzlich zu einer Steuer- und Entnahmeöffnung, eine Einfüllöffnung auf. Diese Einfüllöffnung ist vorzugweise unabhängig von den beiden anderen Öffnungen im Magazin eingebracht. Dies bietet den Vorteil, dass die Einfüllöffnung sehr gross sein kann um ein Einfüllen zu erleichtern. Die Einfüllöffnung wird nach einem Einfüllen eines Pulvers verschlossen, vorzugsweise mit einer Folie versiegelt. Ist das Medikamentenmagazin als Ringmagazin mit mehreren kreisförmig darin angeordneten Medikamentenkammern gestaltet, so befinden sich Steuer- und Entnahmeöffnung jeder Kammer beabstandet zueinander vorzugsweise auf einer Seite des Magazins, während die Einfüllöffnung auf einer gegenüberliegenden Seite des Magazins angeordnet ist. Damit ist auch ein Öffnen der Steuer- und Entnahmeöffnung bei einer Entnahme von einer Verschlussfolie unabhängig. Vorzugsweise weist ein Medikamentenmagazin eine Vielzahl von Medikamentenkammern und darin eingebrachten Einzeldosen auf. Die Anzahl liegt vorzugsweise in einem Bereich von 1 bis 100 oder bis 200 Einzeldosen, bevorzugt in einem Bereich von 1-60, beispielsweise zwischen 7-180 oder 14-150, z.B. 30-120, 45-100, 30, 90, 60, 120. Für Inhalationsgeräte liegt eine Maximalanzahl von Einzeldosen aus Handlichkeits-und Therapie-Gründen vorzugsweise bei 60.

Ein Inhalator mit einem solchen Medikamentenmagazin ist sehr vielfältig in seiner Verwendung. Nebst den unterschiedlichen Abstimmungsmöglichkeiten und gezielten und sehr präzisen Dosierungsmöglichkeiten, insbesondere deren Wiederholbarkeit, bietet der Inhalator auch die Möglichkeit mit den unterschiedlichsten Mengen und Arten von Medikament verwendet zu werden. Dabei kann relativ einfach ein anderes Medikamentenmagazin verwendet werden, beispielsweise mit veränderter, beispielsweise vergrössert oder verkleinert, Kapazität einer Medikamentenkammer. Wird eine Entnahmeöffnung durch das Medikamentenmagazin selber genau definiert ist, kann eine daran anschliessende (Unterdruck-)Öffnung im Venturisaugrohr auch grösser gestaltet sein, ohne die Entnahmeöffnung und dadurch die Entleerungsströmung zu beeinflussen. Dadurch ist ein Inhalator auch mit unterschiedlichen Medikamentenmagazinen, z.B. solchen mit unterschiedlich gestalteten Entnahmeöffnungen verwendbar. Dadurch, dass lediglich eine einzelne Entnahmeöffnung, inhalatorseitig angekoppelt werden muss, ist die übrige Gestaltung des Inhalator, aber auch die der Medikamentenkammer, in Bezug auf einen Entnahmemechanismus im wesentlichen unabhängig vom Medikamentenmagazin und vice versa. Insbesondere kann eine Medikamentenkammer sehr langgestreckt sein, einen Winkel aufweisen etc., und eine Steueröffnung entsprechend an unterschiedlichen Orten im Magazin angeordnet sein. Dies ist möglich, da die Steueröffnung nicht direkt mit der Anordnung des Venturirohres verbunden ist, und insbesondere die im Venturirohr produzierte Antriebsströmung und die durch die Ankopplung der Unterdruckströmung an das Magazin gebildete Entleerungsströmung einen unterschiedlichen Ursprung haben.

Im erfindungsgemässen Inhalator ist im wesentlichen jede Art eines Öffnungsmechanismus möglich: eine Medikamentenkammer kann vor einer Medikamentenentnahme durch ein Anstechen, ein Abschaben oder ein Peelen einer die Medikamentenkammer verschliessende Siegelfolie geöffnet werden. Ein Medikamentenmagazin ist dazu entsprechend gestaltet, vorzugsweise derart, dass die Entnahme- und Steueröffnung, welche auch als Kontrollöffnungen bezeichnet werden können, nicht durch den Öffnungsmechanismus beeinflusst werden. Sie werden insbesondere nicht durch Öffnungen in einer Siegelfolie selber bzw. durch einen solche Öffnungen verursachender Öffnungsmechanismus der Vorrichtung beeinflusst. Dies kann beispielsweise dadurch erreicht werden, dass eine Siegelfolie vor Entnahme des Medikaments gänzlich von den Kontrollöffnungen entfernt wird, oder aber dass eine Öffnung in einer Siegelfolie so gross ist, dass ihr Einfluss auf die Kontrollöffnungen vernachlässigbar ist.

In einer Ausführungsform der Magazins wird ein Medikament in eine Vorkammer entleert, wobei die Vorkammer eine entsprechende Steuer- und Entnahmeöffnung aufweist, welche zur Entnahme des Medikaments an eine Unterdruckströmung angeschlossen wird. Dazu kann ein Medikament mit einer Siegelfolie in einer eigentlichen Medikamentenkammer eingeschlossen sein, welche Folie vorzugsweise durch ein Öffnungsmittel, welches in die Kammer selber eingebaut sein kann, geöffnet wird. Bei dieser Ausführungsform ist die Einfüllöffnung im wesentlichen auf derselben Seite wie die Kontrollöffnungen angeordnet. Die Vorkammer kann dabei integraler Bestandteil der Medikamentenkammer bzw. des Medikamentenmagazins sein, oder aber als integraler, an einen Luftkanal geformter Teil sein. Der Vorteil der ersten Variante ist, dass bei jeder Medikamentenentnahme eine neue, unbenutzte Vorkammer zur Verfügung steht.

In einer bevorzugten Ausführungsform des Inhalators sind zwei Medikamentenkammern über ein gemeinsames Mundstück miteinander verbunden. Damit kann eine Verdoppelung der Anzahl Medikamenteneinzeldosen erreicht werden. Dazu sind die einzelnem Medikamentenkammern der beiden Magazine vorzugsweise versetzt angeordnet. Es ist aber auch möglich bei einer Entnahme je eine Kammer beider Magazine zu öffnen und ein Medikament aus beiden Kammern zu entnehmen. Dies ist insbesondere vorteilhaft falls zwei Medikamente eingenommen werden müssen, die nicht gemeinsam lagerbar sind, oder falls jedes zweite Mal eine doppelte Menge eines Medikaments oder eine Medikamentenmischung erforderlich ist.

Zur Herstellung eines Medikamentenmagazins und gegebenenfalls auch für gewisse Teile einer Vorrichtung werden bevorzugt pharmazeutisch zugelassene Materialien verwendet. Als Folien können beispielsweise Mehrschichtfolien verwendet werden, welche als Peelfolie, Stechfolie oder zum Abschaben geeignet sind. Mehrschichtfolien weisen meist eine Schicht PE, PP oder PVC und eine Aluminiumschicht auf. Je nach Anforderung ist eine Folie, stabiler oder reissfester gestaltet, was vorzugsweise durch den Einbau einer PET Schicht realisiert wird. In bevorzugten Materialkombinationen weist eine Aussenschicht einer Folie dasselbe Material auf, wie beispielsweise ein Spritzgussmagazin. Dadurch ist ein Siegeln bzw. ein Verschweissen der unterschiedlichen Elemente eines Medikamentenmagazins mit denselben Materialien möglich. Es ist auch möglich Siegellack, z.B. Heissiegellack, zu verwenden. Dadurch sind mehr Materialkombinationen der einzelnen Elemente möglich. Ein Siegellack wird dabei beispielsweise auf eine Folie und/oder auf ein zu verschliessendes Magazin aufgebracht. Es ist auch möglich, dass eine Folie direkt eine Lack-Siegelschicht aufweist. Die Stärke der Siegelung wird bevorzugt ebenfalls auf die Verwendung optimiert, so dass beispielsweise eine peelbare Folie dicht, aber ablösbar an einem zu versiegelnden Element haftet.

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino] ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel AC-1 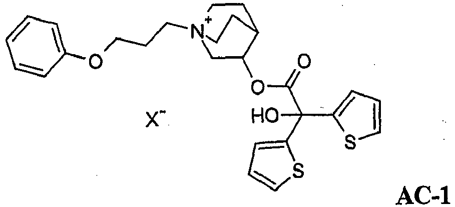 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 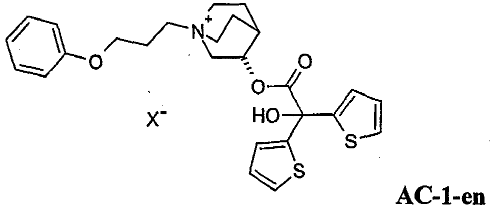 enthalten, worin X⁻ die vorstehend genannten Bedeutungen aufweisen kann. Weiterhin bevorzugte Anticholinergika sind ausgewählt aus den Salzen der Formel **AC-2** 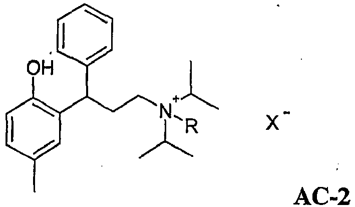 worin R entweder Methyl oder Ethyl bedeuten und worin X⁻ die vorstehend genannte Bedeutungen aufweisen kann. In einer alternativen Ausführungsform kann die Verbindung der Formel **AC-2** auch in Form der freien Base **AC-2-base** vorliegen.

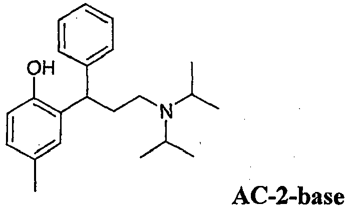

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{(4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]-amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2 buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino)-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkomalkaloide: Dihydroergotamin, Ergotamin.

Für die Inhalation kommen Arzneimittel, Arzneimittelformulierungen und - mischungen mit den o.g. Wirkstoffen in Betracht, sowie deren Salze, Ester sowie die Kombination dieser Wirkstoffe, Salze und Ester.

Im Folgenden ist die Erfindung anhand von Beispielen näher dargestellt. Dabei zeigt
Fig. 1 einen Ausschnitt aus einem Pulverinhalator mit dem Entnahmeprinzip,
Fig. 2 das Entnahmeprinzip bei vereinfachter Darstellung,
Fig. 3 ein Medikamentenmagazin als Ringscheibe,
Fig 4 das Medikamentenmagazin mit geöffneter Medikamentenkammer,
Fig. 5 das gesamte Medikamentenmagazin,
Fig. 6 eine vermasste Darstellung des Medikamentenmagazins gemäss Fig. 3-5,
Fig. 7 eine Medikamentenkammer für unterschiedliche Öffnungsmechanismen,
Fig. 8 ein doppeltes Ringmagazin mit kombiniertem Luftkanal,
Fig. 9 ein doppeltes Ringmagazin mit getrennten Luftkanälen,
Fig. 10 einen Blister mit Innenstruktur und Vorkammer als Medikamentenmagazin,
Fig. 11 zwei Ausführungsformen eines Blisters mit Innenstruktur.

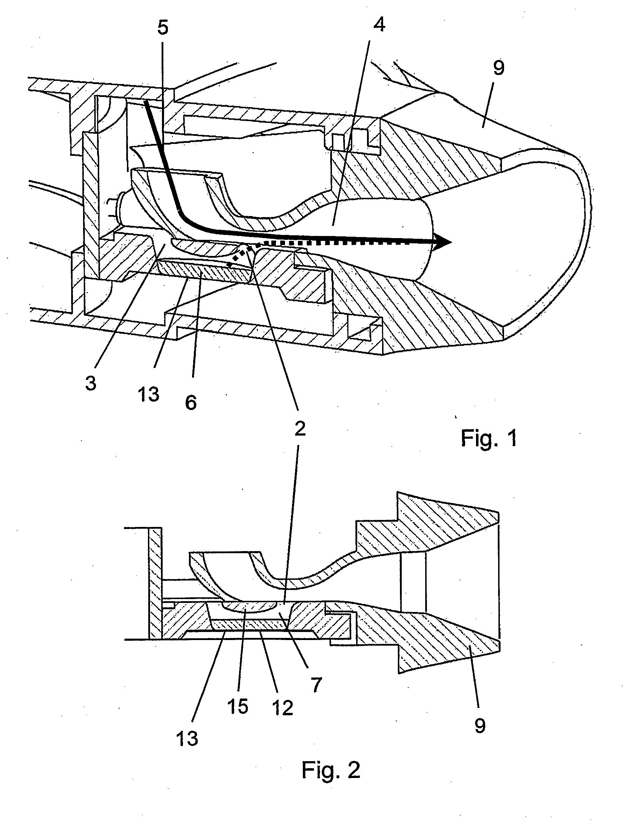

In Figur 1 und Figur 2 ist das Entnahmeprinzip für ein in einer Medikamentenkammer befindliches, ein Medikament enthaltendes, Pulver 6, gezeigt, wie es in einem Pulverinhalator anwendbar ist. Die Medikamentenkammer ist als Kavität 7 in einem, vorzugsweise durch Spritzgiessen oder Thermoformen hergestellten, Kunststoffteil ausgebildet. Die Medikamentenkammer weist an einer oberen Seite eine Entnahmeöffnung 2 auf, welche direkt an einen Luftkanal 4 angeschlossen ist. Der Luftkanal ist als Venturirohr ausgeführt. Bei Benutzung des Inhalators wird durch Saugen an einem Mundstück 9 im Luftkanal eine Antriebsströmung 5 und im Bereich der Verengung des Luftkanals eine Unterdruckströmung erzeugt (Venturieffekt). Das in der Kavität befindliche Pulver wird durch die Entnahmeöffnung in den Luftkanal 4 herausgesogen und gelangt mit der Antriebsströmung 5 in Richtung Mundstück zu einer inhalierenden Person. Durch den Venturieffekt herrscht an der Entnahmeöffnung der grösste Druckunterschied und die grösste Geschwindigkeit im Luftstrom. Dies garantiert ein effizientes Ablösen eines Medikamentes von seinem Träger (Dispergieren des Pulvers). Die Medikamentenkammer weist zudem auf ihrer oberen Seite, derselben Seite wie die Entnahmeöfnung, eine Steueröffnung 3 auf, durch welche Luft in und durch die Kavität strömt und eine Entleerungsströmung bildet. Ein Teil dieser Entleerungsströmung strömt über das Pulver 6 hinweg und kann mit einem in der Kavität befindlichen Verwirbelungselement 15 einen zusätzlichen Venturieffekt zwischen dem Verwirbelungselement und dem Pulver bzw. dem Kavitätboden bilden. Das Verwirbelungselement kann zudem Strukturen aufweisen, z. B. in die Medikamentenkammer weisende Vorsätze, welche eine Verwirbelung der Luft und des Pulvers verstärken.

Jedes einzelne dieser Massnahmen und Effekte erhöht die Turbulenzbildung in der Kavität und damit die Dispergierung. Allfällige Agglomerationen im Pulver vermögen damit ebenfalls gelöst werden. Auch die Form der Entnahmeöffnung, sowie das Zusammentreffen der Unterdruck- und Entleerungsströmung beeinflussen die Dispersion des Pulvers.

Die Medikamentenkammer weist auf der, der oberen Seite gegenüber liegenden unteren Seite eine Einfüllöffnung 13 auf. Die Einfüllöffnung 13 erstreckt sich im wesentlichen über die gesamte untere Fläche der Kavität und wird, nachdem das Pulver in die Kavität gefüllt wurde, mit einer Siegelfolie 12 verschlossen.

Eine Medikamentenentnahme kann mit diesem Entnahmeprinzip sehr fein und vielseitig auf unterschiedlichste Medikamente, Dosierungen und Personen-bedürfnisse angepasst werden. Dies ist durch eine Variation und Anpassung der Entleerungsströmung, die durch mehrere Parameter beeinflusst wird, möglich: Der Luftkanaldurchmesser über der Entnahmeöffnung, die Abmessungen der Entnahmeöffnung, die Gestaltung der Kavität, sowie die Abmessungen der Steueröffnung. Die spezielle Gestaltung des Medikamentenmagazins erlaubt auch eine einfache Anpassung desselben an unterschiedliche Mengen, Zusammensetzungen oder Konsistenz von Medikamenten, z. B. durch eine Vergrösserung der Kavität und/oder Veränderung der Öffnungsdurchmesser. Die separate Einfüllöffnung erlaubt es zudem, diese möglichst gross zu gestalten, so dass ein Einfüllen eines Pulvers sehr rasch und ohne auf eine spezielle (kleine) Einfüllöffnung zugeschnitten zu sein müssen. Auch geht durch das direkte Anschliessen einer Entnahmeöffnung an den Luftkanal und an die darin herrschende Unterdruckströmung keinerlei Pulver in längeren Durchführungen oder Leitungen verloren.

Bei Bedarf weist der Luftkanal 4 zusätzliche Nebenstrom- oder Bypassöffnungen auf, die in den Luftkanal münden und nach einer Entnahmeöffnung in Richtung Mundstück 9 versetzt, angeordnet sind. Dadurch kann eine benötigte Saugkraft verringert oder aber auch Querströmungen im Luftkanal geschaffen werden zum zusätzlichen Dispergieren eines darin befindlichen Pulvers.

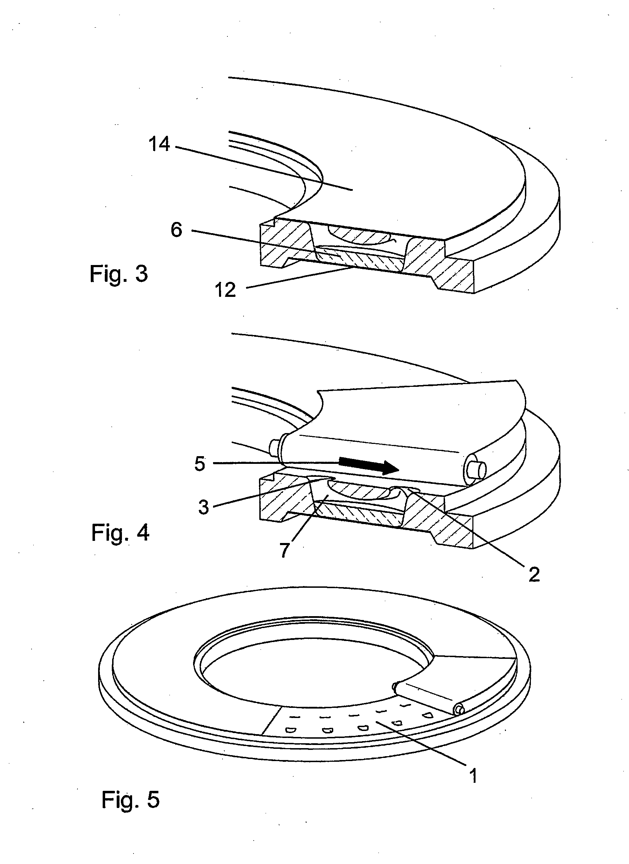

Figur 5 zeigt ein als Ringmagazin 1 ausgebildetes Mehrdosis-Medikamentenmagazin 1, wie es beispielsweise in einem scheibenförmigen Inhalator verwendbar ist. Beim Ringmagazin 1 ist die obere Siegelfolie von fünf Medikamentenkammern abgelöst. Die Aufsicht zeigt jeweils eine Entnahme- und eine Steueröffnung pro Medikamentenkammer. Ein solches Ringmagazin kann einteilig beipielsweise durch Spritzgiessen hergestellt werden. Die obere Siegelfolie wird als erstes aufgesiegelt. Nach dem Befüllen wird die Kavität durch die untere Siegelfolie verschlossen. Beispiele für bevorzugte Spritzgussmaterialien sind PE, PP und PVC.

In den **Figuren 3 und 4** ist jeweils eine Schrägansicht in eine aufgeschnittene Medikamentenkammer gezeigt Figur 3 zeigt eine gefüllte Medikamentenkammer, in welchem sich Pulver 6 in der Kavität 7 befindet. Entnahme- und Steueröffnung sind durch die obere Siegelfolie 14 abgedeckt und werden durch diese luft- und feuchtigkeitsdicht abgeschlossen. Die untere Einfüllöffnung, wovon jede Kavität vorzugsweise ebenfalls eine eigene hat, ist durch eine untere Siegelfolie 12 verschlossen. Figur 4 zeigt dieselbe Schrägansicht einer Medikamentenkammer nach ihrem Gebrauch. Die obere Siegelfolie 14 wurde abgelöst. Der Luftstrom 5 über der Entnahmeöffnung wird durch eine Kanalverengung geführt (siehe Figur 1 und 2), wodurch ein Unterdruck über dieser Öffnung entsteht. Die Dimensionierung der Entnahme- und Steueröffnung ist vorzugsweise so gestaltet, dass eine möglichst kontinuierliche Entleerung der Kavität erreicht wird.

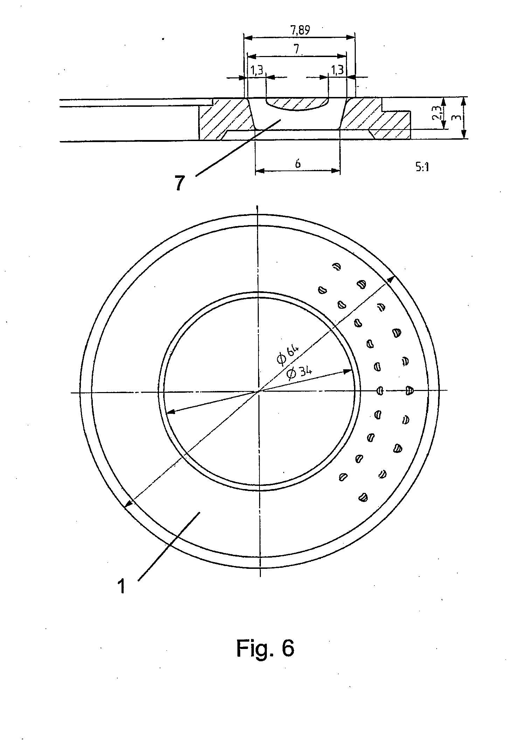

In Figur 6 sind ein Querschnitt durch eine Kavität, sowie eine Aufsicht auf ein Ringmagazin vermasst gezeigt, wie sie beispielsweise für ein Pulvervolumen von ca. 10mm³ und 30 Kavitäten, d.h. Einzeldosen, in einem Ringmagazin realisierbar sind.

Die Menge des Füllvolumens einer Einzeldosis kann dabei relativ einfach variiert werden, indem die Tiefe und/oder Länge der Kavität verändert wird. Da nur die Entnahmeöffnung an eine Unterdruckströmung angeschlossen werden muss, kann die Steueröffnung diesbezüglich an einer praktisch beliebigen Stelle im Magazin angeordnet sein. Sie müsste also nicht benachbart zur Entnahmeöffnung auf derselben Seite des Ringmagazins, sondern könnte beispielsweise auch auf einem inneren Durchmesser des Magazins angeordnet sein.

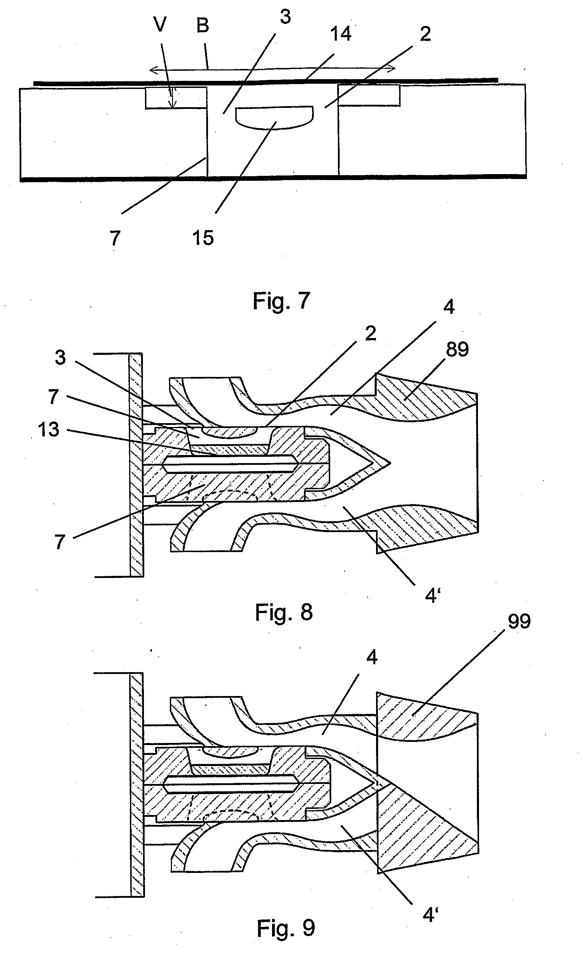

In **Figur 7** ist ein Schnitt durch den Aufbau einer Medikamentenkammer schematisch gezeigt, wie sie gestaltet sein kann, um mit unterschiedlichen Öffnungsmechanismen, wie Peelen, Schaben oder Stechen geöffnet zu werden, ohne dass dabei die Kontrollöffnungen, d.h. die Steuer- und Entnahmeöffnung, beeinflusst werden. Figur 7 zeigt eine Kavität 7 mit voneinander beanstandeten Entnahme- und Steueröffnung 2, 3. Die Öffnungen sind in eine Vertiefung eingelassen. Die Vertiefungshöhe V und Breite B kann nun auf den jeweiligen Öffnungsmechanismus eingestellt werden. Werden die Öffnungen durch ein Abpeelen einer zuvor aufgebrachten Siegelfolie geöffnet, so ist die Vertiefungshöhe V vorzugsweise Null und die Vertiefungsbreite B (bzw. Fläche) entspricht der Kammerbreite. Bei bekannten Anstechverfahren würde eine Siegelfolie durchstochen und die Grösse und Form einer Entnahmeöffnung und einer Steueröffnung würden durch die Stechwerkzeuge bestimmt. Diese sind in der Regel ungenau und eine Drosselströmung wäre nicht definiert und insbesondere nicht reproduzierbar. Um nun ein Stechen als Öffnungsmechanismus verwenden zu können, und dabei von den genau definierten Öffnungen, wie sie bei einer Herstellung durch Spritzgusstechnik machbar sind, zu profitieren, wird die Vertiefungshöhe V>0 und die Vertiefungsbreite B grösser als die Kammerbreite gewählt. Dadurch geschieht ein Stechen unabhängig von der eigentlichen Entnahme- und Steueröffnung. Das Stechwerkzeug ist dabei so gewählt, dass die Folie über einen oder mehrere grössere Bereiche, als die Fläche der Entnahme- und Steueröffnung aufgestochen wird, derart, dass ein Einfluss der Stechöffnung(en) im Verhältnis zu den Kontrollöffnungen vernachlässigbar klein ist.

In den **Figuren 8 und 9** ist jeweils ein Schnitt durch ein Doppel-Medikamentenmagazin, z. B. Ringmagazin dargestellt. Das Magazin entspricht im wesentlichen zwei Ringmagazinen gemäss der Figur 3, welche gespiegelt mit ihrer Rückseite, d. h. der Seite mit den Einfüllöffnungen 13, aneinander anliegen und aneinander festgemacht oder auch nur aneinandergepresst sind. Die beiden Teile des Magazins sind dabei gegeneinander verschoben, derart, dass die einzelnen Medikamentenkammern 7 nicht genau einander gegenüber liegen (versetzte Kammer gestrichelt eingezeichnet). Damit kann ein Mehrdosisinhalator von beispielsweise einem 30-Dosen in einen bevorzugten 60-Dosen Inhalator umgewandelt werden. In Figur 8 münden zwei Luftkanäle 4, 4', welche jeweils zu einem oberen und unteren Teil des Magazins gehören, in dasselbe Mundstück 89. Mundstück und Luftkanäle sind dabei einstückig aufgebaut. Aufgrund der versetzten Anordnung der Medikamentenkammern 7 (in der Figur gestrichelt eingezeichnet) kann selbst bei geöffneten Kammern jeweils nur aus einer Kammer Pulver entnommen werden. Der nicht im Einsatz stehende Luftkanal 4' kann für eine zusätzliche Luftzufuhr verwendet werden (Bypass). Es ist aber auch möglich, die Kammern nicht versetzt anzuordnen, damit aus zwei Kammern, beispielsweise zwei unterschiedliche Medikamente, entnommen werden können. In Figur 9 ist ein Mundstück 99 gezeigt, welches die beiden Luftkanäle 4,4' unterteilt, so dass keine Luft durch einen nicht ausgewählten Kanal ins Mundstück 99 gelangen kann. Dabei ist das Mundstück beispielsweise von einem oberen Teil zu einem unteren Teil des Magazins drehbar.

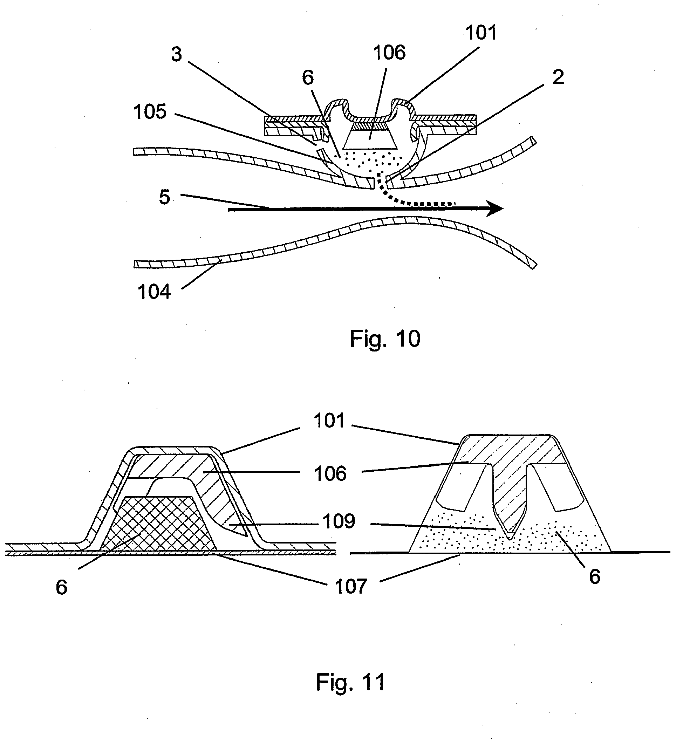

In der **Figur 10** ist eine weitere Ausführungsform der Erfindung anhand eines Medikamentenmagazins gezeigt, welches als Folienblister mit Innenstruktur 101 gestaltet ist. Diese Innenstruktur 106 weist Öffnungsmittel z.B. in der Form von Stechpunkten oder Schneidkanten auf, mit welchen eine, eine Medikamentenkammer schliessende, Siegelfolie durch Ausdrücken der Innenstruktur geöffnet wird. Die Innenstruktur ist vorzugsweise auch so gestaltet, dass sie der Medikamentenkammer eine gewisse Stabilität verleiht. Dadurch wird ein darin befindliches Pulver vor mechanischen Einflüssen, von aussen oder insbesondere beim Öffnen der Kammer geschützt. Dies ist bei pulverförmigen Medikamententrägern für Inhalatoren wichtig, da ein Inhalieren oder Dosieren eines kompaktierten Pulvers nicht mehr oder nicht mehr definiert möglich ist.

Der Folienblister 101 weist im Bereich der eigentlichen Medikamentenkammer eine Vorkammer 105 auf. Die Vorkammer 105 weist eine Entnahmeöffnung 2 und eine Steueröffnung 3 auf. Das Ausbringen des Pulvers 6 aus dem Blister geschieht nun bevorzugt wie folgt: Der Inhalt des Blisters wird mit Hilfe der Innenstruktur, vorzugsweise direkt bei einer Ladebewegung eines Inhalators, in die Vorkammer 105 geleert oder eingebracht. Die Vorkammer weist eine Entnahmeöffnung 2 auf, welche als Durchgangsbohrung gestaltet sein kann und mit einem Venturi-Saugrohr 104 verbunden ist. Durch den Venturi-Effekt herrscht an der Durchgangsbohrung der grösste Druckunterschied, welcher es ermöglicht das Pulver 6 dosiert aus der Vorkammer 105 zu ziehen. An dieser Stelle erreicht der Luftstrom 5 die höchste Geschwindigkeit, was ein effizientes Ablösen (Dispergieren) eines Wirkstoffes vom Träger ermöglicht.

Vorzugsweise wird ein Blister mit integrierter Vorkammer produziert, derart, dass wiederum ein Medikamentenmagazin mit Einfüllöffnung, Entnahmeöffnung und Steueröffnung eine Einheit bildet, wobei die Öffnungen je nach Anwendung aufeinander abgestimmt sind. Ein Vorteil dieser Variante ist, dass bei jeder Inhalation eine frische Vorkammer zu Verfügung steht. Eine weitere Variante, in der bei jeder Entnahme auch ein frisches Saugrohr bzw. Teil eines Saugrohres zur Verfügung steht ist, wenn der Blister mit integrierter Vorkammer und Saugrohr geformt wird. Allfällige Medikamentenrückstände an Saugrohrwänden, welche insbesondere gerne direkt im Bereich der Entnahmeöffnung vorkommen, würden somit nächste Entnahmen nicht beeinflussen. In einer weiteren Variante ist die Vorkammer wiederum ein integraler Bestandteil des Saugrohres, jedoch unabhängig vom Folienblister und der eigentlichen Medikamentenkammer.

Zur Veranschaulichung von Blistern mit Innenstruktur 101 sind in der Figur 11 zwei Beispiele vergrössert dargestellt. Die Figur zeigt je eine Medikamentenkammer in gefülltem, unbenutztem Zustand. Dabei wird Pulver 6 von der Innenstruktur 3 zumindest teilweise eingeschlossen und vor mechanischem Einfluss von aussen, insbesondere jedoch vor einem Zusammendrücken beim Ausdrücken der Innenstruktur aus der Medikamentenkammer geschützt. Die Innenstrukturen weisen Stechpunkte oder Schneidkanten 109 auf, mit welchen bei Öffnen der Kammern eine Siegelfolie 107 angestochen bzw. angeschnitten und aufgerissen bzw. -schnitten wird.

Die Innenstruktur kann mit dem Blister verbunden sein, z.B. an ihrer Rückseite mit einer Folie versiegelt sein, um je nach Lage des Blisters nicht in die Vorkammer zu fallen. Ist jedoch die Vorkammer ein integraler Bestandteil des Blisters bzw. jeder einzelnen darin befindlichen Medikamentenkammer, so ist diese Massnahme gegebenenfalls nicht nötig. Eine solche Innenstruktur kann auch als Verwirbelungselement dienen auch oder insbesondere falls es frei in der Vorkammer liegt. Die Innenstruktur kann durch eine Entleerungsströmung, die durch die Vorkammer führt, für zusätzliche Turbulenzen sorgen und damit zusätzlich zur Deagglomeration und Dispersion des Pulvers beitragen. Die Innenstruktur ist vorzugsweise sehr offen gestaltet, so dass ein Luftstrom möglichst von vielen Seiten in und durch die Innenstruktur dringen kann. Dies trägt zu einer möglichst vollständigen Entleerung einer Medikamentenkammer und zusätzlichen Luftverwirbelungen bei.

Innenstruktur, Vorkammer und Saugrohr werden vorzugsweise durch Spritzgiessen eines Kunststoffes hergestellt. Es ist jedoch auch möglich einzelne Teile davon durch einen Thermoformprozess oder im Falle von Innenstrukturen diese aus einer Folie, z. B. einer Metallfolie, zu stanzen und formen.

## Patentansprüche

1. Medikamenten-Ausgabevorrichtung enthaltend ein Mundstück (9) und einen damit verbundenen Luftkanal (4), sowie ein Medikamentenmagazin mit mindestens einer, ein Medikament beinhaltende Medikamentenkammer mit einer Entnahmeöffnung (2), wobei im Luftkanal (4) eine Antriebsströmung erzeugbar ist, die eine Unterdruckströmung erzeugt,
**dadurch gekennzeichnet, dass**
der Luftkanal (4) als Venturirohr ausgeführt ist, wobei der die Unterdruckströmung verursachende engste Bereich des Luftkanals (4) an eine durch das Medikamentenmagazin vorgegebene Entnahmeöffnung (2) angeschlossen ist, und die Entnahmeöffnung (2) mit einer Steueröffnung (3) zusammenwirkt, zur Bildung einer Entleerungsströmung durch die Steueröffnung (3) via Entnahmeöffnung (2) und die Entnahmeöffnung (2) und Steueröffnung (3) im Medikamentenmagazin integriert und vordefiniert sind.

2. Medikamenten-Ausgabevorrichtung gemäss Anspruch 1, wobei in der Kavität der Medikamentenkammer ein Verwirbelungselement (15) angeordnet ist, derart dass ein zusätzlicher Venturieffekt zwischen dem Verwirbelungselement (15) und einem in der Medikamentenkammer enthaltenen Pulver und/oder dem Kavitätboden erzeugt werden kann.

3. Medikamenten-Ausgabevorrichtung gemäss einem der vorangehenden Ansprüche, wobei eine Bypassöffnung (8) im Luftkanal (4) vorgesehen ist zur Bildung einer Bypassströmung, welche Bypassöffnung nach der Entnahmeöffnung (2) in Richtung des Mundstücks (9) angeordnet ist.

4. Medikamenten-Ausgabevorrichtung gemäss einem der vorhergehenden Ansprüche, mit einer Drosselanordnung zur Variation einer Menge pro Zeit entnommenen Medikaments, wobei die Drosselanordnung die Steueröffnung (3) und die Entnahmeöffnung (2) beinhaltet.

5. Medikamenten-Ausgabevorrichtung gemäss Anspruch 4, wobei die Drosselanordnung die innere Gestaltung der Medikamentenkammer und/oder die Gestaltung des an die Entnahmeöffnung anschliessenden Luftkanals beinhaltet.

6. Medikamenten-Ausgabevorrichtung gemäss einem der vorhergehenden Ansprüche, wobei das Medikamentenmagazin (1) eine Mehrzahl von Medikamentenkammern mit mindestens einer Entnahmeöffnung (2) zur Entnahme eines Medikaments enthält.

7. Medikamenten-Ausgabevorrichtung gemäss einem der vorhergehenden Ansprüche, wobei die Medikamentenkammer eine Einfüllöffnung (13) enthält, wobei die Entnahmeöffnung (2), die Steueröffnung (3) und die Einfüllöffnung (13) vorzugsweise voneinander beabstandet sind.

8. Medikamenten-Ausgabevorrichtung gemäss einem der Ansprüche 6 bis 7, wobei das Medikamentenmagazin ringförmig ist und mehrere Medikamentenkammern ringförmig im Medikamentenmagazin angeordnet sind.

9. Medikamenten-Ausgabevorrichtung gemäss Anspruch 7, wobei in der Medikamentenkammer ein Verwirbelungselement (7) angeordnet ist und das Verwirbelungselement (7) durch mindestens einen Teil der Medikamentenkammerwand gebildet wird.

10. Medikamenten-Ausgabevorrichtung gemäss einem der Ansprüche 6 bis 9, **dadurch gekennzeichnet, dass** die Medikamenten-Ausgabevorrichtung als Mehrdosispulverinhalator ausgebildet ist.

11. Medikamenten-Ausgabevorrichtung gemäss Anspruch 10, wobei das Medikamentenmagazin 60 Medikamentenkammern mit Medikamenteneinzeldosen enthält.

12. Medikamentenmagazin für eine Medikamenten-Ausgabevorrichtung gemäss einem der Ansprüche 1 bis 11, **dadurch gekennzeichnet, dass** das Medikamentenmagazin (1) eine Mehrzahl von Medikamentenkammern mit mindestens einer Öffnung (2) zur Entnahme eines Medikaments enthält, und die Medikamentenkammer eine Einfüllöffnung (13) enthält, und die Entnahmeöffnung (2) mit einer Steueröffnung (3) in Wirkverbindung ist, wobei die Entnahmeöffnung (2) an eine Unterdruckströmung anschliessbar ist zur Bildung einer Entleerungsströmung durch die Steueröffnung (3) via Entnahmeöffnung (2), und wobei Entnahmeöffnung (2) und Steueröffnung (3) im Medikamentenmagazin integriert und vordefiniert sind.

13. Medikamentenmagazin gemäss Anspruch 12, wobei Einfüllöffnung (13), Entnahmeöffnung (2) und Steueröffnung (3) voneinander beabstandet angeordnet sind.

14. Medikamentenmagazin gemäss Anspruch 12 oder 13, wobei das Medikamentenmagazin ringförmig ist und mehrere Medikamentenkammern ring-förmig im Medikamentenmagazin angeordnet sind.

15. Medikamentenmagazin gemäss einem der Ansprüche 12 bis 14, wobei die Einfüllöffnung (13)
- in einer der Entnahme- und Steueröffnung (2,3) gegenüberliegenden Seite des Medikamentenmagazins oder
- auf derselben Seite des Medikamentenmagazins wie die Entnahme- und Steueröffnung (2,3)
angeordnet ist.

## Claims

1. Medicament dispensing device comprising a mouthpiece (9) and an air channel (4) connected therewith, as well as a medicament magazine with at least one medicament chamber with a removal opening (2), the medicament chamber containing a medicament, wherein a drive current comprising a vacuum flow can be generated in the air channel (4),
**characterised in that**
the air channel (4) is formed as a Venturi tube, wherein the most narrow region of the air channel (4) that produces the vacuum flow, is connected to a removal opening (2) provided by the medicament magazine, the removal opening (2) cooperates with a control opening (3), to form an emptying current through the control opening (3) via the removal opening (2), and the removal opening (2) and control opening (3) are integrated and predefined in the medicament magazine.

2. Medicament dispensing device according to claim 1, wherein in the cavity of the medicament chamber a turbulence element (15) is located, such that an additional Venturi effect between the turbulence element (15) and a powder contained in the medicament chamber and/or the base of the cavity is induced.

3. Medicament dispensing device according to one of the preceding claims,
wherein a bypass opening (8) is provided in the air channel (4) for forming a bypass flow, the bypass opening being arranged downstream of the removal opening (2) in the direction of the mouthpiece (9).

4. Medicament dispensing device according to one of the preceding claims, having a throttle arrangement for varying the amount of medicament removed per unit of time, wherein the throttle arrangement comprises the control opening (3) and the removal opening (2).

5. Medicament dispensing device according to claim 4, wherein the throttle arrangement comprises the inner configuration of the medicament chamber and/or the configuration of the air channel connected to the removal opening.

6. Medicament dispensing device according to one of the preceding claims,
wherein the medicament magazine (1) comprises a plurality of medicament chambers, each having at least one removal opening (2) for the removal of a medicament.

7. Medicament dispensing device according to one of the preceding claims,
wherein the medicament chamber comprises a fill opening (13), wherein the removal opening (2), the control opening (3) and the fill opening (13) preferably are spaced from one another.

8. Medicament dispensing device according to one of the claims 6 to 7, wherein the medicament magazine is annular and a plurality of medicament chambers are arranged in a ring in the medicament magazine.

9. Medicament dispensing device according to claim 7, wherein a turbulence element (7) is arranged in the medicament chamber and the turbulence element (7) is formed by at least a part of the wall of the medicament chamber.

10. Medicament dispensing device according to one of the claims 6 to 9, wherein the medicament dispensing device is configured as a multi-dose powder inhaler.

11. Medicament dispensing device according to claim 10, wherein the medicament magazine comprises 60 medicament chambers with single doses of medicament.

12. Medicament magazine for a medicament dispensing device according to one of the claims 1 to 11, **characterized in that** the medicament magazine (1) comprises a plurality of medicament chambers, each having at least one opening (2) for the removal of a medicament, and the medicament chamber has a fill opening (13), and the removal opening (2) is in operative connection with a control opening (3), wherein the removal opening (2) can be attached to a vacuum flow in order to form an emptying current through the control opening (3) via the removal opening (2), and wherein the removal opening (2) and the control opening (3) are integrated and predefined in the medicament magazine.

13. Medicament magazine according to claim 12, wherein the fill opening (13), the removal opening (2) and the control opening (3) are arranged spaced from one another.

14. Medicament magazine according to claim 12 or 13, wherein the magazine is annular and a plurality of medicament chambers are arranged in a ring in the medicament magazine.

15. Medicament magazine according to one of claims 12 to 14, wherein the fill opening (13):
- is arranged in a side of the medicament magazine opposite the removal and control opening (2, 3) or
- on the same side of the medicament magazine as the removal and control opening (2, 3).

## Revendications

1. Dispositif d'émission de médicament contenant une pièce d'embouchure (9) à laquelle est relié un canal d'air (4) ainsi qu'un magasin à médicament doté d'au moins une chambre à médicament qui contient un médicament et qui est dotée d'une ouverture de prélèvement (2),
un écoulement d'entraînement qui forme un écoulement en dépression pouvant être établi dans le canal d'air (4),
**caractérisé en ce que**
le canal d'air (4) est configuré en tube Venturi,
**en ce que** la partie la plus étroite du canal d'air (4) qui provoque l'écoulement en dépression est raccordée à une ouverture de prélèvement (2) prédéterminée par le magasin à médicament,
**en ce que** l'ouverture de prélèvement (2) coopère avec une ouverture de commande (3) qui forme un écoulement de vidange par l'ouverture de commande (3) via l'ouverture de prélèvement (2) et
**en ce que** l'ouverture de prélèvement (2) et l'ouverture de commande (3) sont intégrées et prédéfinies dans le magasin à médicament.

2. Dispositif d'émission de médicament selon la revendication 1, dans lequel un élément de tourbillonnage (15) est disposé dans la cavité de la chambre à médicament de telle sorte qu'un effet Venturi supplémentaire puisse être formé entre l'élément de tourbillonnage (15), une poudre contenue dans la chambre à médicament et/ou le fond de la cavité.

3. Dispositif d'émission de médicament selon l'une des revendications précédentes, dans lequel une ouverture de dérivation (8) est prévue dans le canal d'air (4) pour former un écoulement de dérivation, l'ouverture de dérivation étant disposée en aval de l'ouverture de prélèvement (2) en direction de la pièce d'embouchure (9).

4. Dispositif d'émission de médicament selon l'une des revendications précédentes, présentant un ensemble d'étranglement qui modifie la quantité de médicament prélevée par unité de temps, l'ensemble d'étranglement contenant l'ouverture de commande (3) et l'ouverture de prélèvement (2).

5. Dispositif d'émission de médicament selon la revendication 4, dans lequel l'ensemble d'étranglement contient la configuration interne de la chambre à médicament et/ou la configuration du canal d'air qui se raccorde à l'ouverture de prélèvement.

6. Dispositif d'émission de médicament selon l'une des revendications précédentes, dans lequel le magasin (1) à médicament contient plusieurs chambres à médicament qui présentent au moins une ouverture de prélèvement (2) permettant de prélever un médicament.

7. Dispositif d'émission de médicament selon l'une des revendications précédentes, dans lequel la chambre à médicament contient une ouverture de remplissage (13), l'ouverture de prélèvement (2), l'ouverture de commande (3) et l'ouverture de remplissage (13) étant de préférence disposées à distance les unes des autres.

8. Dispositif d'émission de médicament selon l'une des revendications 6 à 7, dans lequel le magasin à médicament est de forme annuaire et dans lequel plusieurs chambres à médicament sont disposées en anneau dans le magasin à médicament.

9. Dispositif d'émission de médicament selon la revendication 7, dans lequel un élément de tourbillonnage (7) est disposé dans la chambre à médicament, l'élément de tourbillonnage (7) étant formé par au moins une partie de la paroi de la chambre à médicament.

10. Dispositif d'émission de médicament selon l'une des revendications 6 à 9, **caractérisé en ce que** le dispositif d'émission de médicament est configuré comme inhalateur de poudre multidose.

11. Dispositif d'émission de médicament selon la revendication 10, dans lequel le magasin à médicament contient 60 chambres à médicament dotées de doses unitaires de médicament.

12. Magasin à médicament pour dispositif d'émission de médicament selon l'une des revendications 1 à 11, **caractérisé en ce que**
le magasin (1) à médicament contient plusieurs chambres à médicament dotées d'au moins une ouverture (2) de prélèvement d'un médicament,
**en ce que** la chambre à médicament contient une ouverture de remplissage (13) et l'ouverture de prélèvement (2) coopère avec une ouverture de commande (3),
**en ce que** l'ouverture de prélèvement (2) peut être raccordée à un écoulement en dépression pour former un écoulement de vidange par l'ouverture de commande (3) et via l'ouverture de prélèvement (2) et
**en ce que** l'ouverture de prélèvement (2) et l'ouverture de commande (3) sont intégrées et prédéfinies dans le magasin à médicament.

13. Magasin à médicament selon la revendication 12, dans lequel l'ouverture de remplissage (13), l'ouverture de prélèvement (2) et l'ouverture de commande (3) sont disposées à distance les unes des autres.

14. Magasin à médicament selon les revendications 12 ou 13, le magasin à médicament étant de forme annulaire et plusieurs chambres à médicament étant disposées en anneau dans le magasin à médicament.

15. Magasin à médicament selon l'une des revendications 12 à 14, dans lequel l'ouverture de remplissage (13) est disposée sur un côté du magasin à médicament situé face à l'ouverture de prélèvement et à l'ouverture de commande (2, 3) ou est disposé du même côté du magasin à médicament que l'ouverture de prélèvement et l'ouverture de commande (2, 3).
